# EUROPEAN PATENT SPECIFICATION

(11) **EP 1 988 905 B1**
(45) Date of publication and mention of the grant of the patent: **04.06.2014**
(21) Application number: 07737738.0
(22) Date of filing: 27.02.2007
(51) Int. Cl.: A61K 31/558, A61K 31/5575

(54) **METHOD AND COMPOSITION FOR TREATING CHRONIC OBSTRUCTIVE PULMONARY DISEASE**
VERFAHREN UND ZUSAMMENSETZUNG ZUR BEHANDLUNG VON CHRONISCH OBSTRUKTIVER LUNGENERKRANKUNG
PROCÉDÉ ET COMPOSITION POUR LE TRAITEMENT DE LA MALADIE PULMONAIRE OBSTRUCTIVE CHRONIQUE

(30) Priority: 28.02.2006 US 776943 P
(43) Date of publication of application: 12.11.2008
(73) Proprietor: Sucampo AG, 6300 Zug (CH)
(72) Inventor: UENO, Ryuji, Montgomery, Maryland 20854 (US); KUNO, Sachiko, Montgomery, Maryland 20854 (US)
(74) Representative: Adam, Holger
(86) International application number: PCT/JP2007/054127
(87) International publication number: WO 2007/102446

(56) References cited:
- EP-A- 0 430 552
- EP-A- 0 978 284

## Description

### TECHNICAL FIELD

The present invention describes a method and composition for treating chronic obstructive pulmonary disease in a mammalian subject.

### BACKGROUND ART

Chronic Obstructive Pulmonary Disease (COPD) is a disease state characterized by airflow limitation that is not fully reversible. The airflow limitation is usually both progressive and associated with an abnormal inflammatory response of the lungs to noxious particles or gases. COPD is a comprehensive term frequently used to describe two conditions of fixed airways disease, chronic bronchitis and emphysema, but excludes asthma (reversible airflow limitation).

The most important risk factor for COPD is cigarette smoking. Pipe, cigar, and other types of tobacco smoking popular in many countries are also risk factors for COPD.

Other causes of COPD include occupational dusts and chemicals (vapors, irritants, and fumes) when the exposures are sufficiently intense or prolonged, indoor air pollution from biomass fuel used for cooking and heating in poorly vented dwellings or outdoor air pollution, adds to the lungs' total burden of inhaled particles, although its specific role in causing COPD is not well understood. Passive exposure to cigarette smoke also contributes to respiratory symptoms and COPD. Respiratory infections in early childhood are associated with reduced lung function and increased respiratory symptoms in adulthood (Global Initiative for Chronic Obstructive Lung Disease, POCKET GUIDE TO COPD DIAGNOSIS, MANAGEMENT, AND PREVENTION, A Guide for Health Care Professionals, UPDATED JULY, 2005).

Physicians report that current therapies provide only symptomatic relief and would welcome a treatment that can alter the development of COPD, either slowing the progressive loss of lung function or, more importantly, reversing established disease itself. However, none of the emerging therapies has yet to translate these concepts into clinical benefit. Many agents in various therapeutic classes are being investigated. However, these are not expected to address the key unmet need of mitigating or reversing progressive loss of lung function.

Prostaglandins (hereinafter, referred to as PG(s)) are members of class of organic carboxylic acids, which are contained in tissues or organs of human or other mammals, and exhibit a wide range of physiological activity. PGs found in nature (primary PGs) generally have a prostanoic acid skeleton as shown in the formula (A):

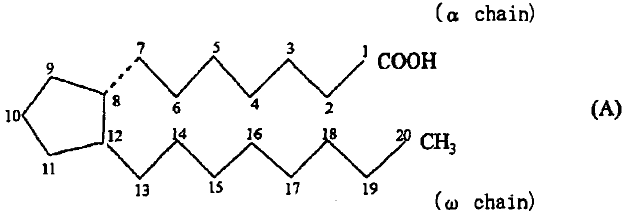

On the other hand, some of synthetic analogues of primary PGs have modified skeletons. The primary PGs are classified into PGAs, PGBs, PGcs, PGDs, PGEs, PGFs, PGGs, PGHs, PGIs and PGJs according to the structure of the five-membered ring moiety, and further classified into the following three types by the number and position of the unsaturated bond at the carbon chain moiety:
Subscript 1: 13,14-unsaturated-15-OH
Subscript 2: 5,6- and 13,14-diunsaturated-15-OH
Subscript 3: 5,6-, 13,14-,and 17,18-triunsaturated-15-OH.

Further, the PGFs are classified, according to the configuration of the hydroxyl group at the 9-position, into α type (the hydroxyl group is of an α-configuration) and β type (the hydroxyl group is of a β-configuration).

PGE₁ and PGE₂ and PGE₃ are known to have vasodilation, hypotension, gastric secretion decreasing, intestinal tract movement enhancement, uterine contraction, diuretic, bronchodilation and anti ulcer activities. PGF_{1α}, PGF_{2α} and PGF_{3α} have been known to have hypertension, vasoconstriction, intestinal tract movement enhancement, uterine contraction, lutein body atrophy and bronchoconstriction activities.

Some 15-keto (i.e., having oxo at the 15-position instead of hydroxy)-PGs and 13,14-dihydro (i.e., having single bond between the 13 and 14-position)-15-keto-PGs are known as the substances naturally produced by the action of enzymes during the metabolism of primary PGs.

U.S. Patent No. 5,254,588 to Ueno et al. describes that some 15-keto-PG compounds are useful for the treatment of a pulmonary dysfunction.

U.s. Patent No. 5,362,751 to Ueno et al.describes that some 15-keto-PGE compounds are useful as a tracheobronchodilator.

U.S. Patent No.6,197,821 to Ueno et al. describes that some 15-keto-PGE compounds are an antagonist for endothelin which is considered to have a relation to hypertension, Buerger disease, asthema, eyegrounds diseases, and the like.

U.S. Patent No.7,064,148 and U.S. Patent Publication No. 2003/0166632 to Ueno et al. describes prostaglandin compound opens and activates chloride channels, especially ClC channels, more especially ClC-2 channel.

It is not known how the specific bicyclic compound acts on COPD.

### SUMMARY OF THE INVENTION

An object of the present invention to provide a method for the preparation of a medicament for the treatment of chronic obstructive pulmonary disease in a mammalian patient. In another aspect, an object of the present invention is to provide a pharmaceutical composition for use in the treatment of chronic obstructive pulmonary disease in a mammalian patient. The present inventor conducted an intensive study and found that specific bicyclic compound is useful for the treatment of COPD, which have resulted in the completion of the present invention.

Namely, the present invention describes a method for treating chronic obstructive pulmonary disease in a mammalian subject, which comprises administering an effective amount of a bicyclic compound represented by Formula (I): 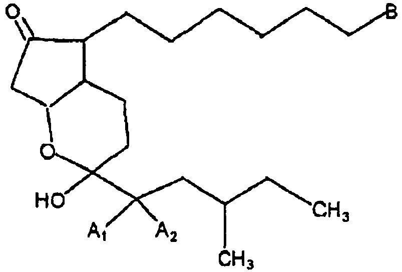 wherein A₁ and A₂ are the same or different halogen atoms and
B is -CH₃, -CH₂OH, -COCH₂OH -COOH, or its pharmaceutically acceptable salts, ethers, esters or amides and/or its tautomer to a subject in need thereof.

The present invention also provide a pharmaceutical composition comprising an effective amount of a bicyclic compound represented by the above formula (I) and/or its tautomer for treating chronic obstructive pulmonary disease in a mammalian subject.

The present invention further provides use of a bicyclic compound represented by the above formula (I) and/or its tautomer, for manufacturing a pharmaceutical composition for treating chronic obstructive pulmonary disease in a mammalian patient.

### BRIEF DESCRIPTION OF THE DRAWINGS

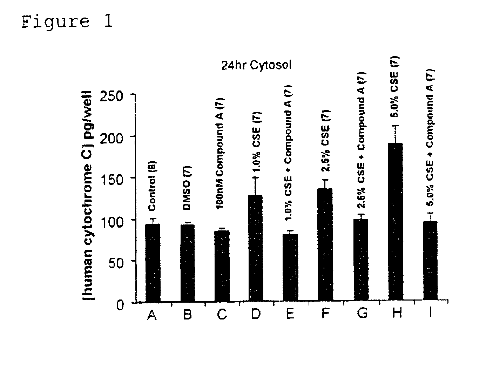

Fig. 1 depicts a graph showing Cytochrome c translocation from A549 mitochondria after 24 hours CSE treatment in the presence or absence of 100 nM Compound A. A549 cells were grown to confluence and treated with CSE at 1% (E), 2.5% (H) and 5%(K) for 24 hours. After that, cytochrome c translocation was measured. Neither 0.1% DMSO (B) (vehicle for Compound A) nor Compound A (C) alone significantly affected cytosolic cytochrome c. CSE caused a significant increase in cytochrome c translocation in a dose dependent manner. At all doses of CSE, 100 nM Compound A (E, G, I) protected against the cytochrome c translocation induced by CSE. Data are expressed as mean ± SEM pg/well, n, the number of wells per point is shown above each bar. Data are expressed as pg/well cytochrome c. Each well contained 1.5 x 10⁵ cells.

### DETAILED DESCRIPTION OF THE INVENTION

The bicyclic compound used in the present invention is represented by formula (I): 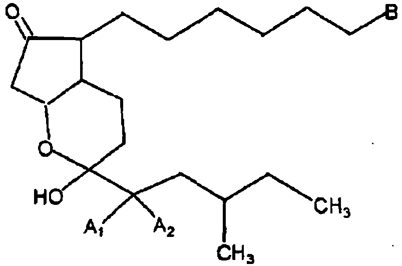 wherein A₁ and A₂ are the same or different halogen atoms and
B is -CH₃, -CH₂OH, -COCH₂OH -COOH, or its pharmaceutically acceptable salts, ethers, esters or amides.

The term "halogen" is used conventionally to include fluorine, chlorine, bromine, and iodine atoms. Particularly preferable halogen atoms for A₁ and A₂ are fluorine atoms.

Suitable "pharmaceutically acceptable salts" include conventionally used non-toxic salts, for example a salt with an inorganic base such as an alkali metal salt (such as sodium salt and potassium salt), an alkaline earth metal salt (such as calcium salt and magnesium salt), an ammonium salt; or a salt with an organic base, for example, an amine salt (such as methylamine salt, dimethylamine salt, cyclohexylamine salt, benzylamine salt, piperidine salt, ethylenediamine salt, ethanolamine salt, diethanolamine salt, triethanolamine salt, tris(hydroxymethylamino)ethane salt, monomethyl- monoethanolamine salt, procaine salt and caffeine salt), a basic amino acid salt (such as arginine salt and lysine salt), tetraalkyl ammonium salt and the like. These salts may be prepared by a conventional process, for example from the corresponding acid and base or by salt interchange.

Examples of the ethers include alkyl ethers, for example, lower alkyl ethers such as methyl ether, ethyl ether, propyl ether, isopropyl ether, butyl ether, isobutyl ether, t-butyl ether, pentyl ether and 1-cyclopropyl ethyl ether; and medium or higher alkyl ethers such as octyl ether, diethylhexyl ether, lauryl ether and cetyl ether; unsaturated ethers such as oleyl ether and linolenyl ether; lower alkenyl ethers such as vinyl ether, allyl ether; lower alkynyl ethers such as ethynyl ether and propynyl ether; hydroxy (lower) alkyl ethers such as hydroxyethyl ether and hydroxyisopropyl ether; lower alkoxy (lower)alkyl ethers such as methoxymethyl ether and 1-methoxyethyl ether; optionally substituted aryl ethers such as phenyl ether, tosyl ether, t-butylphenyl ether, salicyl ether, 3,4-di-methoxyphenyl ether and benzamidophenyl ether; and aryl(lower)alkyl ethers such as benzyl ether, trityl ether and benzhydryl ether.

Examples of the esters include aliphatic esters, for example, lower alkyl esters such as methyl ester, ethyl ester, propyl ester, isopropyl ester, butyl ester, isobutyl ester, t-butyl ester, pentyl ester and 1-cyclopropylethyl ester; lower alkenyl esters such as vinyl ester and allyl ester; lower alkynyl esters such as ethynyl ester and propynyl ester; hydroxy (lower) alkyl ester such as hydroxyethyl ester; lower alkoxy (lower) alkyl esters such as methoxymethyl ester and 1-methoxyethyl ester; and optionally substituted aryl esters such as, for example, phenyl ester, tolyl ester, t-butylphenyl ester, salicyl ester, 3,4-di-methoxyphenyl ester and benzamidophenyl ester; and aryl(lower)alkyl ester such as benzyl ester, trityl ester and benzhydryl ester.

The amide of B means a group represented by the formula -CONR'R", wherein each of R' and R" is hydrogen atom, lower alkyl, aryl, alkyl- or aryl-sulfonyl, lower alkenyl and lower alkynyl, and include for example lower alkyl amides such as methylamide, ethylamide, dimethylamide and diethylamide; arylamides such as anilide and toluidide; and alkyl- or aryl-sulfonylamides such as methylsulfonylamide, ethylsulfonyl-amide and tolylsulfonylamide.

Preferred embodiment comprises a bicyclic compound of formula (I) in which A₁ and A₂ are fluorine atoms, and B is -COOH.

The bicyclic compound of this invention exists as a bicyclic form in a solid state, but partially forms a tautomer of the above compound when dissolved in a solvent. In the absence of water, compounds represented by formula (I) exist predominantly in the form of the bicyclic compound. In aqueous media, it is believed that hydrogen bonding occurs between, for example, the ketone at the C-15 position, thereby hindering bicyclic ring formation. In addition, it is believed that the halogen atoms at the C-16 position promote bicyclic ring formation. The tautomerism between the hydroxy at the C-11 position and the keto moiety at the C-15 position, shown below, is especially significant in the case of compounds having a 13,14 single bond and two fluorine atoms at the C-16 position.

According to the present invention, said "tautomer" of the compound of formula (I), for example, a mono-cyclic tautomer having a keto group at the C-15 position and halogen atoms at the C-16 position, may also be used for the treatment.

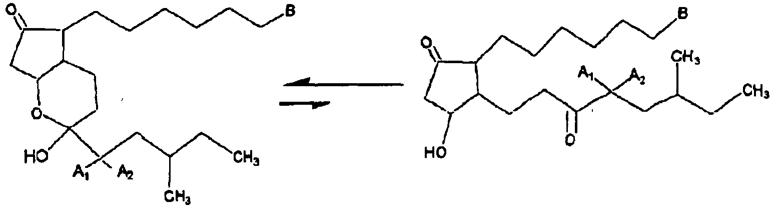

A preferred compound according to the invention in its monocyclic form can be named as 13,14-dihydro-15-keto-16,16-difluoro-18(S)-methyl-PGE₁, according to conventional prostaglandin nomenclature.

The compound used in the present invention may be prepared by the method disclosed in US No. 5, 739, 161.

According to the present invention, a mammalian subject may be treated by the instant invention by administering the compound used in the present invention. The subject may be any mammalian subject including a human. The compound may be applied systemically or topically. Usually, the compound may be administered by oral administration, intranasal administration, inhalational administration, intravenous injection (including infusion), subcutaneous injection, intra rectal administration, transdermal administration and the like.

The dose may vary depending on the strain of the animal, age, body weight, symptom to be treated, desired therapeutic effect, administration route, term of treatment and the like. A satisfactory effect can be obtained by systemic or topical administration 1-4 times per day or continuous administration at the amount of 0.00001-500µg/kg per day, preferably 0.0001-100µg/kg, more preferably 0.001-10µg/kg.

The compound may preferably be formulated in a pharmaceutical composition suitable for administration in a conventional manner. The composition may be those suitable for oral administration, intranasal administration, inhalational administration, injection or perfusion as well as it may be an external agent, suppository or pessary.

The composition of the present invention may further contain physiologically acceptable additives. Said additives may include the ingredients used with the present compounds such as excipient, diluent, filler, resolvent, lubricant, adjuvant, binder, disintegrator, coating agent, cupsulating agent, ointment base, suppository base, aerozoling agent, emulsifier, dispersing agent, suspending agent, thickener, tonicity agent, buffering agent, soothing agent, preservative, antioxidant, corrigent, flavor, colorant, a functional material such as cyclodextrin and biodegradable polymer, stabilizer. The additives are well known to the art and may be selected from those described in general reference books of pharmaceutics.

The amount of the above-defined compound in the composition of the invention may vary depending on the formulation of the composition, and may generally be 0.000001-10.0%, more preferably 0.00001-5.0%, most preferably 0.0001-1%.

Examples of solid compositions for oral administration include tablets, troches, sublingual tablets, capsules, pills, powders, granules and the like. The solid composition may be prepared by mixing one or more active ingredients with at least one inactive diluent. The composition may further contain additives other than the inactive diluents, for example, a lubricant, a disintegrator and a stabilizer. Tablets and pills may be coated with an enteric or gastroenteric film, if necessary. They may be covered with two or more layers. They may also be adsorbed to a sustained release material, or microcapsulated. Additionally, the compositions may be capsulated by means of an easily degradable material such gelatin. They may be further dissolved in an appropriate solvent such as fatty acid or its mono, di or triglyceride to be a soft capsule. Sublingual tablet may be used in need of fast-acting property.

Examples of liquid compositions for oral administration, intranasal administration or inhalational administration include emulsions, solutions, suspensions, syrups and elixirs and the like. Said composition may further contain a conventionally used inactive diluents e.g. purified water or ethyl alcohol. The composition may contain additives other than the inactive diluents such as adjuvant e.g. wetting agents and suspending agents, sweeteners, flavors, fragrance and preservatives.

The composition of the present invention may be in the form of spraying composition which contains one or more active ingredients and may be prepared according to a known method.

Examples of the intranasal preparations may be aqueous or oily solutions, suspensions or emulsions comprising one or more active ingredients. For the administration of an active ingredient by inhalation, the composition of the present invention may be in the form of suspension, solution or emulsion which can provide aerosol or in the form of powder suitable for dry powder inhalation. The composition for inhalational administration may further comprise a conventionally used propellant.

Examples of the injectable compositions of the present invention for parenteral administration include sterile aqueous or non-aqueous solutions, suspensions and emulsions. Diluents for the aqueous solution or suspension may include, for example, distilled water for injection, physiological saline and Ringer's solution.

Non-aqueous diluents for solution and suspension may include, for example, propylene glycol, polyethylene glycol, vegetable oils such as olive oil, alcohols such as ethanol and polysorbate. The composition may further comprise additives such as preservatives, wetting agents, emulsifying agents, dispersing agents and the like. They may be sterilized by filtration through, e.g. a bacteriaretaining filter, compounding with a sterilizer, or by means of gas or radioisotope irradiation sterilization. The injectable composition may also be provided as a sterilized powder composition to be dissolved in a sterilized solvent for injection before use.

Another form of the present invention is suppository or pessary, which may be prepared by mixing the active ingredients into a conventional base such as cacao butter that softens at body temperature, and nonionic surfactants having suitable softening temperatures may be used to improve absorbability.

The term "treatment" or "treating" used herein includes any means of control such as prevention, care, relief of the condition, attenuation of the condition and arrest of progression.

As mentioned above, the term "chronic obstructive pulmonary disease" or "COPD" includes a disease state characterized by airflow limitation that is not fully reversible. COPD is a comprehensive term frequently used to describe two conditions of fixed airways disease, chronic bronchitis and emphysema. Accordingly, the present compound is useful for the treatment of COPD including chronic bronchitis and emphysema.

COPD is often associated with exacerbations of symptoms and many exacerbations are caused by infection of the tracheobronchial tree or an increase in air pollution. According to the present invention, the treatment of infection based on or accompanied by COPD by using the pharmaceutical composition of the invention is also provided.

The pharmaceutical composition of the present invention may contain one or more compounds of formula (I) and may further contain one or more pharmacologically active ingredients other than compound of formula (I) as far as they do not contradict the purpose of the present invention.

Further details of the present invention will follow with reference to test examples.

### Example 1

### (Methods)

Guinea pigs were exposed to cigarette smoke using a smoking system (INH06-CIG01A, M.I.P.S. Inc.). Each animal was placed in an exposure holder, and the holder was fixed in an exposure chamber. Cigarette smoke of 30 cigarettes per day (Peace^{®}, Japan Tobacco Inc.) was drawn from a smoke generator into the exposure chamber, 5 days per week, for 25 days (Days 1 to 25). In a sham exposure group, the atmospheric air, instead of cigarette smoke, was drawn into the exposure chamber.

Aqueous solution of Compound A (13,14-dihydro-15-keto-16,16-difluoro-18(S)-methyl-PGE₁) was vaporized using a pressurized nebulizer (LC Plus Nebulizer, Pari GmbH) and inhaled by the animals in a chamber of an inhalation system (SIS-A, Sibata Scientific Technology Ltd.) for 30 minutes from 1 hr before the cigarette smoke exposure.

Specific airway resistance (sRaw) of conscious animals was measured by a double flow plethysmo-graph technique with a respiratory function measurement system (Pulmos-1, M.I.P.S Inc.) on Day 26.

After the sRaw measurements, animals were anesthetized with ketamine (60 mg/kg) and xylazine (8 mg/kg), and the trachea was cannulated. Pulmonary function was measured using a pulmonary function measurement system (Biosystem Manoeuvers, Buxco Electronics, Inc.). The parameters of measurement consisted of residual volume (RV) and forced expiratory volume at 100 msec (FEV₁₀₀).

After the measurement of pulmonary function, the animals were sacrificed by exsanguination under anesthesia and the thorax was opened. Five milliliters of saline were instilled into the lungs through a tracheal cannula, and the lavage fluid was recovered by gentle aspiration. This procedure was repeated and the recovered lavage fluid was combined (10mL in total, bronchoalveolar lavage fluid, BALF). The number of macrophages (monocyte) in the BALF was counted.

### (Results)

As shown in Table 1, specific airway resistance (sRaw) in control group was increased by the cigarette smoke exposure as compared with that in sham exposure group. Compound A significantly inhibited the increase in sRaw induced by the cigarette smoke exposure as compared with the control group.

As shown in Table 2, residual volume (RV) in control group was increased and forced expiratory volume at 100 msec (FEV₁₀₀) was decreased by the cigarette smoke exposure as compared with those in the sham exposure group. Compound A significantly inhibited these changes induced by the cigarette smoke exposure as compared with the control group.

As shown in Table 3, the number of macrophages (monocyte) in bronchoalveolar lavage fluid in control group was increased by the cigarette smoke exposure as compared with that in the sham exposure group. Compound A significantly inhibited the increase in number of macrophages (monocyte) induced by the cigarette smoke exposure as compared with the control group.

**Table 1 Effect of Compound A on specific airway resistance (sRaw) in cigarette smoke-exposed guinea pigs**

| Group | Concentration µg/mL | n | Specific airway resistance (sRaw) |
|---|---|---|---|
| | | | Mean ± S.D., cmH2O · sec |
| Sham-exposure | 0 | 8 | 1.131 ± 0.149 |
| Control (Vehicle) | 0 | 8 | 2.154 ± 0.365^{##} |
| Compound A | 1 | 7 | 1.417 ± 0.226** |
| Compound A | 10 | 7 | 1.383 ± 0.241** |

| | | | |
|---|---|---|---|
| ## p<0.01 Significantly different from sham-exposure group ** p<0.01 Significantly different from control group | | | |

**Table 2 Effects of Compound A on residual volume (RV) and forced expiratory volume at 100 msec (FEV₁₀₀) in cigarette smoke-exposed guinea pigs**

| Group | Concentration µg/mL | n | RV Mean ± S.D., mL | FEV₁₀₀ Mean ± S.D., mL |
|---|---|---|---|---|
| Sham-exposure | 0 | 8 | 2.75 ± 1.45 | 9.87 ± 1.17 |
| Control (Vehicle) | 0 | 8 | 5.44 ± 1.43^{##} | 5.75 ± 3.29^{#} |
| Compound A | 1 | 7 | 3.45 ± 1.07* | 10.38 ± 1.08** |

| | | | | |
|---|---|---|---|---|
| # p<0.05, ## p<0.01 Significantly different from sham-exposure group * p<0.05, ** p<0.01 Significantly different from control group | | | | |

**Table 3 Effect of Compound A on cell count of macrophages (monocyte) in bronchoalveolar lavage fluid in cigarette smoke-exposed guinea pigs**

| Group | Concentration µg/mL | n | Monocyte /Macrophage Mean ± S.D., 10² cells/µL |
|---|---|---|---|
| Sham-exposure | 0 | 8 | 4.52 ± 1.73 |
| Control (Vehicle) | 0 | 8 | 14.42 ± 3.00^{##} |
| Compound A | 1 | 7 | 10.35 ± 2.53* |

| | | | |
|---|---|---|---|
| ^{##} p<0.01 Significantly different from sham-exposure group * p<0.05 Significantly different from control group | | | |

These results show that Compound A is beneficial to treat COPD.

### Example 2

### (Methods)

Smoke from 8 cigarettes was drawn slowly through 100ml of serum free culture medium and the resulting suspension was filtered through 0.20 µm filter. The solution was considered as 100% cigarette smoke extract (CSE). Human lung alveolar type II cells (A549) were grown in 96 well plates for 48 hrs at a final concentration of 1.5 ×10⁵ cells per well. The cells were then treated separately with either 100nM Compound A or 1%, 2.5% and 5% CSE. In other sets, 100nM Compound A was added along with 1%, 2.5% or 5% CSE. All incubations were done at 37°C for 24hrs. After 24hr treatment, the cells were washed with 0-4°C PBS three times. Measurement of cytochrome c that was translocated into the cytosol, a marker of cellular injury, was performed according to the instructions provided with a kit for cytochrome c ELISA assay.

### (Results)

CSE in the range of 1.0%-5% caused a dose dependent increase in cytochrome c release from the mitochondria into the cytosol of the cells (translocation) measured after 24 hours of treatment. As shown in Fig. 1, there was no significant increase in cytochrome c translocation at 1% CSE compared to control without 0.1% DMSO, but the translocation was significant for 2.5% and 5% CSE (P< 0.01 and P< 0.005,respectively). Similar results were seen when compared to the 0.1% DMSO control for 1%, 2.5% and 5% CSE (NS, P<0.01, and P<0.05, respectively). Compound A significantly decreased cytochrome c translocation compared to DMSO control at 1%, 2.5% and 5% CSE (P<0.05, P<002 and P<0.005 respectively). The results demonstrate protective effects of Compound A on alveolar cells.

These results show that Compound A is beneficial to treat COPD.

## Claims

1. Use of a bicyclic compound represented by Formula (I): 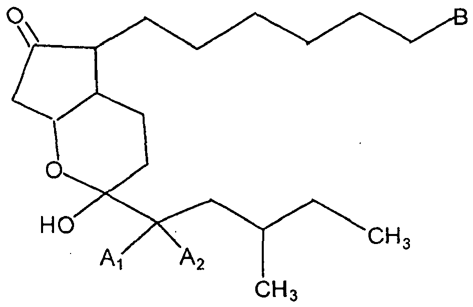
wherein A₁ and A₂ are the same or different halogen atoms and
B is -COOH, including its pharmaceutically acceptable salts, ethers, esters or amides
and/or its tautomer for manufacturing a pharamaceutical composition for the treatment of chronic obstructive pulmonary disease in a mammalian subject.

2. The use as described in claim 1, wherein A₁ and A₂ are fluorine atoms.

3. The use as described in claim 2, wherein B is -COOH.

4. A compound for use in the treatment of chronic obstructive pulmonary disease in a mammalian subject, which comprises administering an effective amount of a bicyclic compound represented by Formula (I): 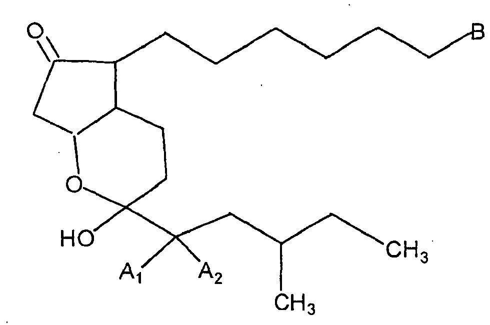
wherein A₁ and A₂ are the same or different halogen atoms and
B is -COOH, including its pharmaceutically acceptable salts, ethers, esters or amides
and/or its tautomer to a subject in need thereof.

5. The compound for use as described in claim 4, wherein A₁ and A₂ are fluorine atoms.

6. The compound for use as described in claim 5, wherein B is -COOH.

7. A pharmaceutical composition for use in treating chronic obstructive pulmonary disease in a mammalian subject, comprising an effective amount of a bicyclic compound represented by Formula (I): 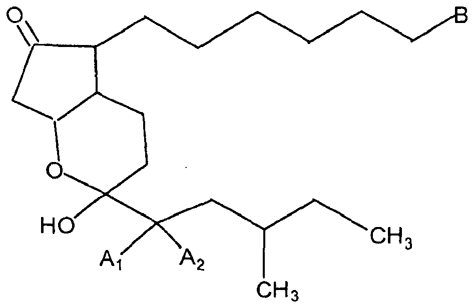
wherein A₁ and A₂ are the same or different halogen atoms and
B is -COOH, including its pharmaceutically acceptable salts, ethers, esters or amides
and/or its tautomer.

8. The composition for use as described in claim 7, wherein A₁ and A₂ are fluorine atoms.

9. The composition for use as described in claim 8, wherein B is -COOH.

## Patentansprüche

1. Verwendung einer bicyclischen Verbindung der Formel (I): 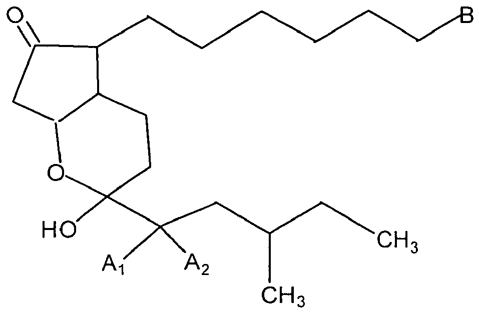
worin A₁ und A₂ gleiche oder verschiedene Halogenatome sind und
B -COOH ist, einschließlich deren pharmazeutisch verträgliche Salze, Ether, Ester oder Amide
und/oder deren Tautomer, zur Herstellung einer pharmazeutischen Zusammensetzung zur Behandlung von chronischobstruktiver Lungenerkrankung bei einem Säugetier.

2. Verwendung nach Anspruch 1, wobei A₁ und A₂ Fluoratome sind.

3. Verwendung nach Anspruch 2, wobei B -COOH ist.

4. Verbindung zur Verwendung bei der Behandlung von chronisch-obstruktiver Lungenerkrankung bei einem Säugetier, die die Verabreichung einer wirksamen Menge einer bicyclischen Verbindung der Formel (I) an einen betroffenen Patienten: 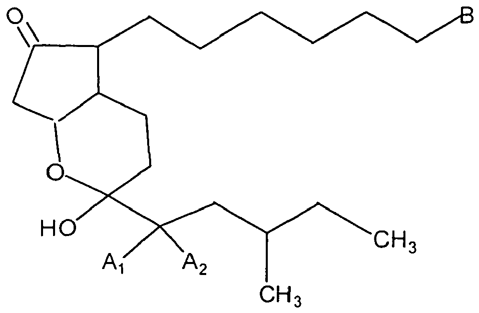
worin A₁ und A₂ die gleichen oder verschiedene Halogenatome sind und
B -COOH ist, einschließlich deren pharmazeutisch verträgliche Salze, Ether, Ester oder Amide
und/oder deren Tautomer, umfasst.

5. Verbindung zur Verwendung nach Anspruch 4, wobei A₁ und A₂ Fluoratome sind.

6. Verbindung zur Verwendung nach Anspruch 5, wobei B -COOH ist.

7. Pharmazeutische Zusammensetzung zur Verwendung bei der Behandlung chronisch-obstruktiver Lungenerkrankung bei einem Säugetier, umfassend eine wirksame Menge einer bicyclischen Verbindung der Formel (I): 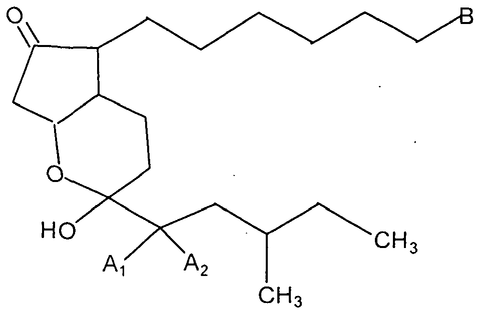
worin A₁ und A₂ die gleichen oder verschiedene Halogenatome sind und
B -COOH ist, einschließlich deren pharmazeutisch verträgliche Salze, Ether, Ester oder Amide
und/oder deren Tautomer.

8. Zusammensetzung zur Verwendung nach Anspruch 7, wobei A₁ und A₂ Fluoratome sind.

9. Zusammensetzung zur Verwendung nach Anspruch 8, wobei B -COOH ist.

## Revendications

1. Utilisation d'un composé bicyclique représenté par la formule (I) : 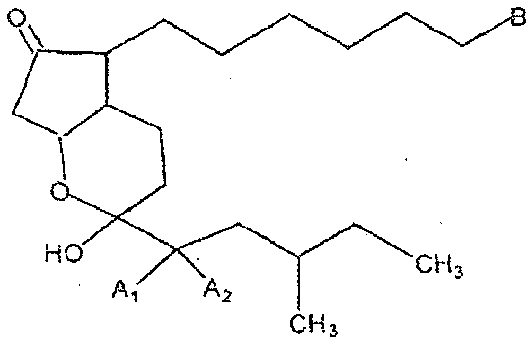
dans laquelle A₁ et A₂ sont des atomes d'halogène identiques ou différents et
B représente un groupe -COOH, y compris ses sels pharmaceutiquement acceptables, éthers, esters ou amides
et/ou son tautomère pour la fabrication d'une composition pharmaceutique destinée au traitement de la bronchopneumopathie chronique obstructive chez un sujet mammifère.

2. Utilisation telle que décrite dans la revendication 1, où A₁ et A₂ sont des atomes de fluor.

3. Utilisation telle que décrite dans la revendication 2, où B représente un groupe -COOH.

4. Composé pour une utilisation dans le traitement de la bronchopneumopathie chronique obstructive chez un sujet mammifère, qui comprend l'administration d'une quantité efficace d'un composé bicyclique représenté par la formule (I) : 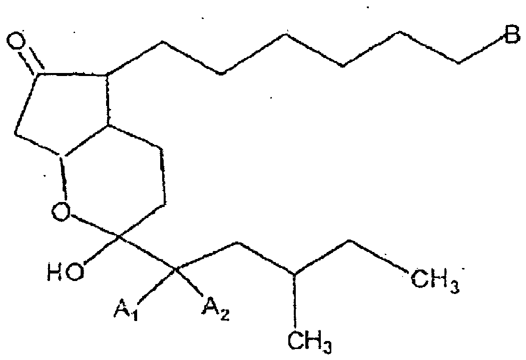
dans laquelle A₁ et A₂ sont des atomes d'halogène identiques ou différents et
B représente un groupe -COOH, y compris ses sels pharmaceutiquement acceptables, éthers, esters ou amides
et/ou son tautomère à un sujet en ayant besoin.

5. Composé pour une utilisation telle que décrite dans la revendication 4, où A₁ et A₂ sont des atomes de fluor.

6. Composé pour une utilisation telle que décrite dans la revendication 5, où B représente un groupe -COOH.

7. Composition pharmaceutique pour une utilisation dans le traitement de la bronchopneumopathie chronique obstructive chez un sujet mammifère, comprenant une quantité efficace d'un composé bicyclique représenté par la formule (I) : 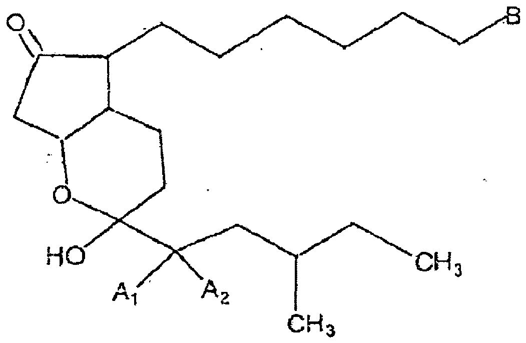
dans laquelle A₁ et A₂ sont des atomes d'halogène identiques ou différents et
B représente un groupe -COOH, y compris ses sels pharmaceutiquement acceptables, éthers, esters ou amides
et/ou son tautomère.

8. Composition pour une utilisation telle que décrite dans la revendication 7, où A₁ et A₂ sont des atomes de fluor.

9. Composition pour une utilisation telle que décrite dans la revendication 8, où B représente un groupe -COOH.
